# EUROPEAN PATENT APPLICATION

(11) **EP 1 040 837 A2**
(43) Date of publication of application: **04.10.2000**
(21) Application number: 00200707.8
(22) Date of filing: 28.02.2000
(51) Int. Cl.: A61K 38/31, A61K 9/06, A61K 9/52, A61K 9/08, A61K 9/10, A61P 27/02

(54) **Medicaments for the treatment of a choroidal neovascularization (CNV) related disorder**

(30) Priority: 26.02.1999 US 258240
(71) Applicant: Erasmus Universiteit Rotterdam, 3015 GE Rotterdam (NL)
(72) Inventor: Kuijpers, Robertus Wilhelmus Aloysius Maria, 3023 BG Rotterdam (NL); van Hagen, Petrus Martinus, 3062 XM Rotterdam (NL); Baarsma, Goitzen Seerp, 3054 CM Rotterdam (NL)
(74) Representative: Ottevangers, Sietse Ulbe

(57) **Abstract**

The invention relates to preparation of medicaments for the treatment of ocular disorders, in particular choroidal neovascularization (CNV) related disorders. The control of ocular neovascularization is regulated by a balance between stimulating and inhibiting growth factors. The anti-angiogenic effect of somatostatin analogues are used to provide a medicament for the treatment of a choroidal neovascularization (CNV) related disorder.

## Description

### FIELD OF THE INVENTION

The invention relates to the preparation of medicaments for the treatment of ocular disorders, in particular choroidal neovascularization (CNV) related disorders.

### BACKGROUND OF THE INVENTION

Age-related maculopathy (ARM) is the major cause of blindness in people over 65 years in the Western world. The prevalence of ARM is up to 14% over age 85. Late stages of ARM, also called age-related macular degeneration (AMD), include geographic atrophy and exudative macular degeneration. The exudative form is characterized by choroidal neovascularization (CNV) and is responsible for 80% of the cases of severe vision loss. These numbers will enlarge because of the increasing average age of the population. In CNV newly formed vessels from the underlying choroid grow beneath the retinal pigment epithelium (RPE) and the retina. Although the morphology of angiogenesis in CNV secondary to AMD has been described in detail, the pathogenesis is still poorly understood, if at all. A balance between a number of stimulating and inhibiting growth factors regulates the control of neovascularization (see: D'Amore PA. "Mechanisms of retinal and choroidal neovascularization", *Invest Ophthalmol Vis Sci*. 1994;35:3974-3979). Vascular endothelial growth factor (VEGF), and endothelial specific mitogen, are regarded as the most important ocular angiogenic factors, specially in ischemic disease. Other regulating growth factors include fibroblast growth factors (FGFs), transforming growth factor β (TGFβ) and insulin-like growth factor I (IGF-I). Most of these growth factors are shown to be upregulated in a diversity of cells (RPE, fibroblasts, capillary endothelial cells) involved in CNV.

Smith *et al*. ("Essential role of growth hormone in ischemia-induced retinal neovascularization", *Science*. 1997;393:93-101) have shown that inhibition of growth hormone (GB), medicated by IGF-I in a transgenic mice model, can inhibit ischemia-induced retinal neovascularization *in vivo*. The release of GH secretion by the pituitary gland is inhibited by somatostatin and somatostatin analogues. Systemic treatment with a somatostatin analogue diminished the level of ocular neovascularization in mice thus indirectly by intervening in the GH secretion of the pituitary gland.

Until now, no suitable methods and medicaments for the selective and/or topical or in situ treatment of CNV related disorders exist.

It is an object of the present invention to provide such a method and medicament.

It is a further object of the present invention to provide new medicaments for CNV related disorders that could not be treated successfully until now.

Such medicaments are of particular relevance in view of the increasing age of the population and the geriatric nature of CNV related disorders.

### SUMMARY OF THE INVENTION

The present inventors have found that the objects of the invention can be achieved by the administration to a patient of compounds that bind to at least one somatostatin receptor, such as sst₁, sst₂, sst₃, sst₄ or sst₅, and preferably to at least the sst₂ receptor, and more preferably to the sst_{2A} receptor.

The present inventors have found that somatostatin or somatostatin analogues directly effect angiogenesis and also have a direct anti-proliferative effect on human retinal endothelial cells. In addition, it was found that sst receptors, such as the sst_{2A} receptor, in choroid and retina of early ARM and non-neovascular AMD are localized similar to normal controls. In eyes with CNV, the sst_{2A} receptor is upregulated in the fibrovascular phase of CNV, as well as in intrachoroidal myofibroblasts. Because of the sst expression in CNV, somatostatin analogues can be employed in a therapy for early stages of CNV in AMD.

### DETAILED DESCRIPTION OF THE INVENTION

In accordance with the present invention, it has been found that a beneficial effect is obtained when compounds that bind to at least one somatostatin receptor in the eye are administered to patients suffering from a CNV related disorder. More in particular, if patients suffering from a CNV related disorder are treated with compounds that bind in the eye to somatostatin receptors, such as sst₁, sst₂, sst₃, sst₄ or sst₅, that the CNV related disorder will diminish or even disappear. Further, inflammation reactions decreased.

Preferably, said compound binds to somatostatin receptors in the eye in the nanomolar range.

In a preferred embodiment of the method of the invention, the said compound binds to a sst₂ receptor, most preferably to a sst_{2A} receptor.

It was known that somatostatin binds with high affinity to 5 subtype receptors (sst₁₋₅). The exact role of a direct receptor mediated effect by somatostatin analogues is, however, still unknown. In particular, information about sst₂ receptor expression in CNV is not available, and until now sst subtype expression is not described in normal human retina.

In general, the ocular disorders to be treated in accordance with the present invention are caused by neovascularization, more in particular by choroidal neovascularization (CNV). Particularly such a disorder is exudative age-related macular degeneration (AMD).

In accordance with the present invention it has been found that the somatostatin receptor binding compounds in the method of treatment of the present invention have a number of beneficial effects. These effects pertain to the stopping of leakage in existing and new ocular vessels and to the inhibition of neovascularization in AMD and DR. The stopping of leakage is relevant to, *e.g*., the treatment of exudates in age related macular degeneration (AMD) and exudates in diabetic retinopathy (DR). The in situ inhibition of neovascularization is relevant to, *e.g*., CNV.

Accordingly, the present invention relates to a medicament for an ocular disorder caused by leakage in existing and new ocular vessels and to neovascularization. This medicament comprises a somatostatin or a somatostatin analogue.

Suitable compounds to be used in the method of the present invention belong to the naturally occurring class of the somatostatins. Somatostatin is a neuropeptide which constitutes a multi gene peptide family with two principal bioactive products, somatostatin-14 and somatostatin-28. It acts on multiple organs including the brain gut, endocrine glands, pancreas, kidneys and the immune system (Reichlin S. Somatostatin (first of two parts). N Engl J Med 1983;309:1495-501, Reichlin S. Somatostatin (second of two parts). N Engl J Med 1983;309:15556-63, Krulich I, Dhariwal AP, McCann SM. Stimulatory and inhibitory effects of purified hypothalamic extracts on growth hormone release from rat pituitary in vitro. Endocrinology 1968;83:783-90, Brazeau P, Vale W, Burgus R, Ling N, Butcher M, Rivier J, Guillemin R. Hypothalamic polypeptide that inhibits the secretion of immunoreactive pituitary growth hormone. Science 1973;179:77-9, Lucey MR. Endogenous somatostatin and the gut. Gut 1986;27:457-67, MehlerPS, Sussman AL, Maman A, Leitner JW, Sussman KE. Role of insulin secretagogues in the regulation of somatostatin binding by isolated rat islets. J Clin Invest 1980;66:1334-8). Somatostatin binds to 5 types of G-protein coupled transmembrane receptors (sst) (Schönbrunn A, Tashjian H Jr. Characterization of functional receptors for somatostatin in ra pituitary cells in culture. J Biol Chem 1978;253:6473-83, Reubi JC, Kvols LK, Krenning EP, Lamberts SWJ. Distribution of somatostatin receptors in normal and tumor tissue. Metabolism 1990;39 Suppl 2:78-81, Patel YC, Greenwood MT, warszynska A, Panetta R, Srikant CB. All five cloned human somatostatin receptors (hSSTR1-5) are functionally coupled to adenylyl cyclase. Biochem Biophys Res Commun 1994;198:605-12). Somatostatin analogues can be used, as well.

More in general, the somatostatin class is a known class of small peptides comprising the naturally occurring somatostatin-14 and analogues having somatostatin related activity, e.g. as disclosed by A.S. Dutta in Small Peptides, Vol. 19, Elsevier (1993). By the term a "somatostatin peptide" or "a somatostatin analogue" as used herein is meant any straight or cyclic polypeptide having a structure based on that of the naturally occurring somatostatin-14 wherein one or more amino acid units have been omitted and/or replaced by one or more other amino radical(s) and/or wherein one or more functional groups have been replaced by one or more other functional groups and/or one or more groups have been replaced by one or several other isosteric groups. In general, the term covers all modified derivatives of the native somatostatin which exhibit a somatostatin related activity, e.g., that bind to at least one somatostatin receptor (sst₁, sst₂, sst₃, sst₄ or sst₅), preferably to at least the sst₂ receptor, even more preferably to at least the sst_{2A} receptor.

The terms a "somatostatin", a "somatostatin peptide" and a "somatostatin analogue" are used within this disclosure as synonyms.

Cyclic, bridged cyclic and straight-chain somatostatin analogues or derivatives are known and have been described together with processes for their production *e.g*. in US-A-4,310,518, US-A-4,235,886 and EP-A-0 001 296, the contents thereof, in particular with respect to the compounds, being incorporated herein by reference.

Preferred somatostatin analogues are, *e.g*., compounds of formula (I). 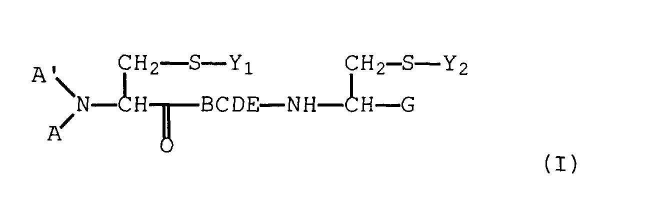 wherein
- A: is C₁₋₁₂alkyl, C₇₋₁₀phenylalkyl or a group of formula RCO-, whereby
(i) R is hydrogen, C₁₋₁₁alkyl, phenyl or C₇₋₁₀phenylalkyl, or
(ii) RCO- is
   a) a D-phenylalanine residue optionally ring-substituted by halogen NO₂, NH₂, OH, C₁₋₃alkyl and/or C₁₋₃alkoxy; or
   b) the residue of a natural or a synthetic α-amino-acid other than defined under a) above, or of a corresponding D-amino acid, or
   c) a dipeptide residue in which the individual amino acid residues are the same or different and are selected from those defined under a) and/or b) above,
the α-amino group of amino acid residues a) and b) and the N-terminal amino group of dipeptide residues c) being optionally mono- or di-C₁₋₁₂alkylated or substituted by C₁₋₈alkanoyl;
- A': is hydrogen or C₁₋₃alkyl,
- Y₁ and Y₂: represent together a direct bond or each of the Y₁ and Y₂ is hydrogen
- B: is -Phe- optionally ring-substituted by halogen, NO₂, NH₂, OH, C₁₋₃alkyl and/or C₁₋₃alkoxy (including pentafluoroalanine), naphthylalanine or pyridylalanine,
- C: is (L)-Trp- or (D)-Trp- optionally a-N-methylated and optionally benzene-ring-substituted by halogen, NO₂, NH₂, OH C₁₋₃alkyl and/or C₁₋₃alkoxy,
- D: is Lys, 4-aminocyclohexylAla or 4-aminocyclohexylGly
- E: is Thr, Ser, Val, Tyr, ILe, Leu or an aminobutyric or aminoisobutyric acid residue
- G: is a group of formula: -COOR₇, -CH₂OR₁₀, -CONR₁₁R₁₂ or 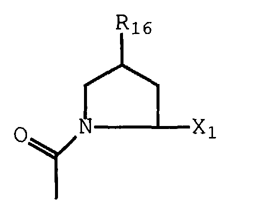 wherein
- R₇: is hydrogen or C₁₋₃alkyl,
- R₁₀: is hydrogen or the residue of a physiologically acceptable, physiologically hydrolysable ester,
- R₁₁: is hydrogen, C₁₋₃alkyl, phenyl or C₇₋₁₀phenyl-alkyl,
- R₁₂: is hydrogen, C₁₋₃alkyl or a group of formula -CH(R₁₃)-X₁,
- R₁₃: is CH₂OH, -(CH₂)₂-OH, -(CH₂)₃-OH, or -CH(CH₃)OH or represents the substituent attached to the a-carbon atom of a natural or synthetic a-amino acid (including hydrogen) and
- X₁: is a group of formula -COOR₇, -CH₂OR₁₀ or -CO-NR₁₄R₁₅ wherein
- R₇ and R₁₀: have the meanings given above,
- R₁₄: is hydrogen or C₁₋₃alkyl and
- R₁₅: is hydrogen, C₁₋₃alkyl, phenyl or C₇₋₁₀phenylalkyl, and
- R₁₆: is hydrogen or hydroxy,
with the proviso that
when R₁₂ is -CH(R₁₃)-X₁ then R₁₁ hydrogen or methyl,
wherein the residues B, D and E have the L-configuration, and the residues in the 2- and 7-position each independently have the (L)- or (D)-configuration,
in free form or in pharmaceutically acceptable salt or complex form.

Individual compounds of formula I suitable in accordance with the present invention are the following somatostatin analogues:

a.	(D)Phe-Cys-Phe-(D)Trp-Lys-Thr-Cys-Tyr-ol
	also known as octreotide

b.	(D)Phe-Cys-Tyr-(D)Trp-Lys-Val-Cys-ThrNH2

c.	(D)Phe-Cys-Tyr-(D)Trp-Lys=Val-Cys-TrpNH2
	also known as vapreotide

d.	(D)Trp-Cys-Phe-(D)Trp-Lys-Thr-Cys-ThrNH2

e.	(D)Phe-Cys-Phe-(D)Trp-Lys-Thr-Cys-ThrNH2

f.	3-(2-(Naphthyl)-(D)Ala-Cys-Tyr-(D)Trp-Lys-Val-Cys-
	ThrNH2
	also knwown as lanreotide

g.	(D)Phe-Cys-Tyr-(D)Trp-Lys-Val-Cys-β-Nal-NH2

h.	3-(2-(Naphthyl)-(D)Ala-Cys-Tyr-(D)Trp-Lys-Val-Cys-β-
	Nal-NH2

i.	(D)Phe-Cys-β-Nal-(D)Trp-Lys-Val-Cys-Thr-NH2

j.	(D)Phe-Cys-Tyr-(D)Trp-Lys-Leu-Cys-Thr-NH2

k.	(D)Phe-Cys-Tyr-(D)Trp-Lys-Cys-Thr-NH2

More preferred compounds of formula (I) are compounds (a) - (k). A highly preferred compound of formula (I) is octreotide.

Compounds of formula (I) may exist e.g. in free form, salt form or in the form of complexes thereof. Acid addition salts may be formed with e.g. organic acids, polymeric acids and inorganic acids. Such acid addition salt forms include e.g. the hydrochlorides and acetates. Complexes are e.g. formed from compounds of the invention on addition of inorganic substances, e.g. inorganic salts or hydroxides such as Ca- and Zn-salts, and/or addition of polymeric organic substances.

According to the invention, the compound binding to somatostatin receptor is preferably administered in the form of a pharmaceutical composition, by any conventional route, in particular enterally, e.g. orally, e.g. in the form of tablets, capsules, drink solutions, emulsions or microemulsion preconcentrates, nasally, pulmonary (by inhalation), parenterally, e.g. in the form of injectable solutions or suspensions, or topically. The compound is preferably administered topically in an ophthalmic preparation, or subcutaneously, e.g. by injection and/or infusion in the eye.

The compound capable of binding to a somatostatin is preferably administered topically to an individual, typically in the form of an ophthalmic liquid preparation (eye drop), in the form of a gel and/or in the form of an ointment.

The compound capable of binding to a somatostatin receptor may also be administered in the eye locally, *e.g*., intravitreally and peribulbarely.

The amount administered is determined taking into account various factors such as the etiology and severity of the disease, and the patient's condition. A somatostatin analogue may be administered, e.g. subcutaneously in a dosage range of about 100 µg to 10 mg per day as a single dose or in divided doses. Thus, octreotide may be administered at a dose of from 0.2 mg to 10 mg twice or three times daily. When administered as a slow release form, such formulation may comprise the somatostatin peptide in a concentration of from 2.0 to 10% by weight. The release period of such a formulation may be from 1 week to about 2 months. Preferably the formulation in slow release form is administered intramuscularly.

It is noted that the use of octreotide in a very specific type of ocular disorder is known from Kuijpers *et al*. ("Treatment of Cystoid Macular Edema with Octreotide", N Engl J Med 338(1998)624-626). In this publication a treatment of a 21 year old patient suffering from idiopathic cystoid macular edema using octreotide is described. However, nothing is taught in this publication with respect to choroidal neovascularization. In addition, this publication is directed to the treatment of a 21-year-old patient and is therefore not in the field of geriatrics. The CNV related disorders which can be treated with the medicaments of the present invention are generally associated with geriatrics.

### BRIEF DESCRIPTION OF THE DRAWINGS

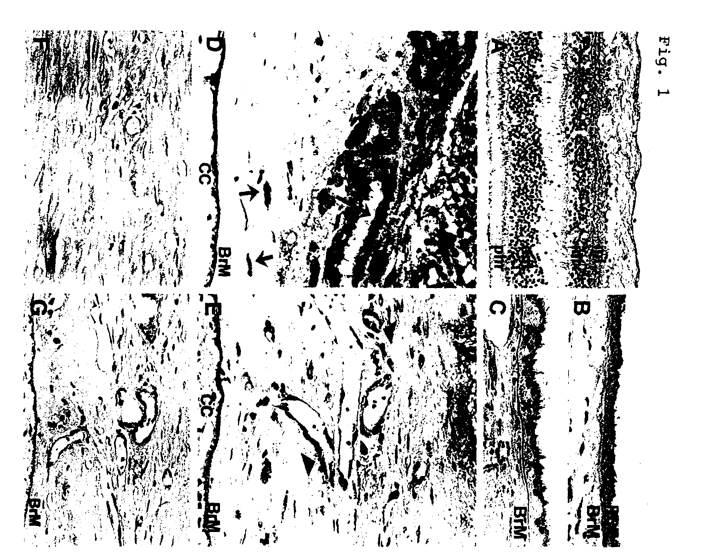

Figure 1. Immunolocalization of sst_{2A} in posterior pole of normal eyes and eyes with different stages of ARM. Immunohistochemistry was performed on paraffin-embedded tissue, and visualized with an alkaline phosphatase detection system using a red chromogen. (A) Positive staining of normal neuroretina, with strong sst_{2A} expression in the inner plexiform layer (ipl) and moderate expression in the outer plexiform layer and the cellular membrane of the inner nuclear layer (inl). (B) Sst_{2A} staining of normal RPE, showing the membranous staining pattern at the apical side. © Sst_{2A} staining of an eye with early ARM, showing negative staining of basal laminar deposits and soft drusen (asterixis). (D,E) Sst_{2A} staining of a mixed fibrovascular and fibrocellular CNV (eye nr 13) in an aye with AMD. Long arrow indicates positive RPE; short arrows indicate positive fibroblast-like cells. Arrowheads indicate positive endothelium of newly formed vessels. Asterixis indicates overlying neuroretina. (F) Sst_{2A} staining of a fibrocellular CNV (eye nr 16) in an eye with AMD, with no staining of endothelial cells and fibroblast-like cells. (G) Negative control staining of CNV (eye nr 13) with peptide blocking. Original magnification (A)x200, (B through H)x400. (onl = outer nuclear layer, phr = photoreceptor layer, RPE = retinal pigment epithelium, BrM = Bruch's Membrane, CC = choriocapillaris).

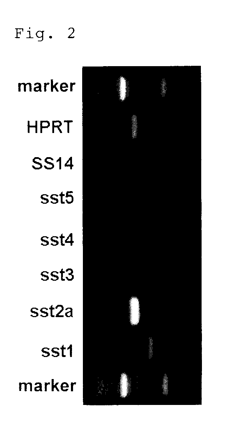

Figure 2. Expression of sst receptor subtype mRNA in the posterior pole of a normal human eye, detected by RT-PCR. Sst₁, sst_{2A} and sst₃ were detected, signals for sst₄ and sst₅ were too low to detect or absent. mRNA for somatostatin (SS14) was also detected. HPRT (hypoxanthine-guanine phosphoribosyl transferase) was used as internal control. (marker = 100 bas pair-marker).

The present invention will be further illustrated by the following non-limiting example.

This example was performed according to the tenets of the Declaration of Helsinki. Enucleation or surgical excision of subfoveal CNVs was performed after obtaining informed consent of the patient.

### EXAMPLE

Patients All eyes were retrieved from the files from the ophthalmic pathology department of the University Hospital of Rotterdam. Sixteen eyes (ten enucleated eyes, four donor eyes and two surgically removed subretinal neovascular membranes) of fifteen patients with different stages of ARM were used for immunohistochemistry. The description of each eye is given in Table 1.

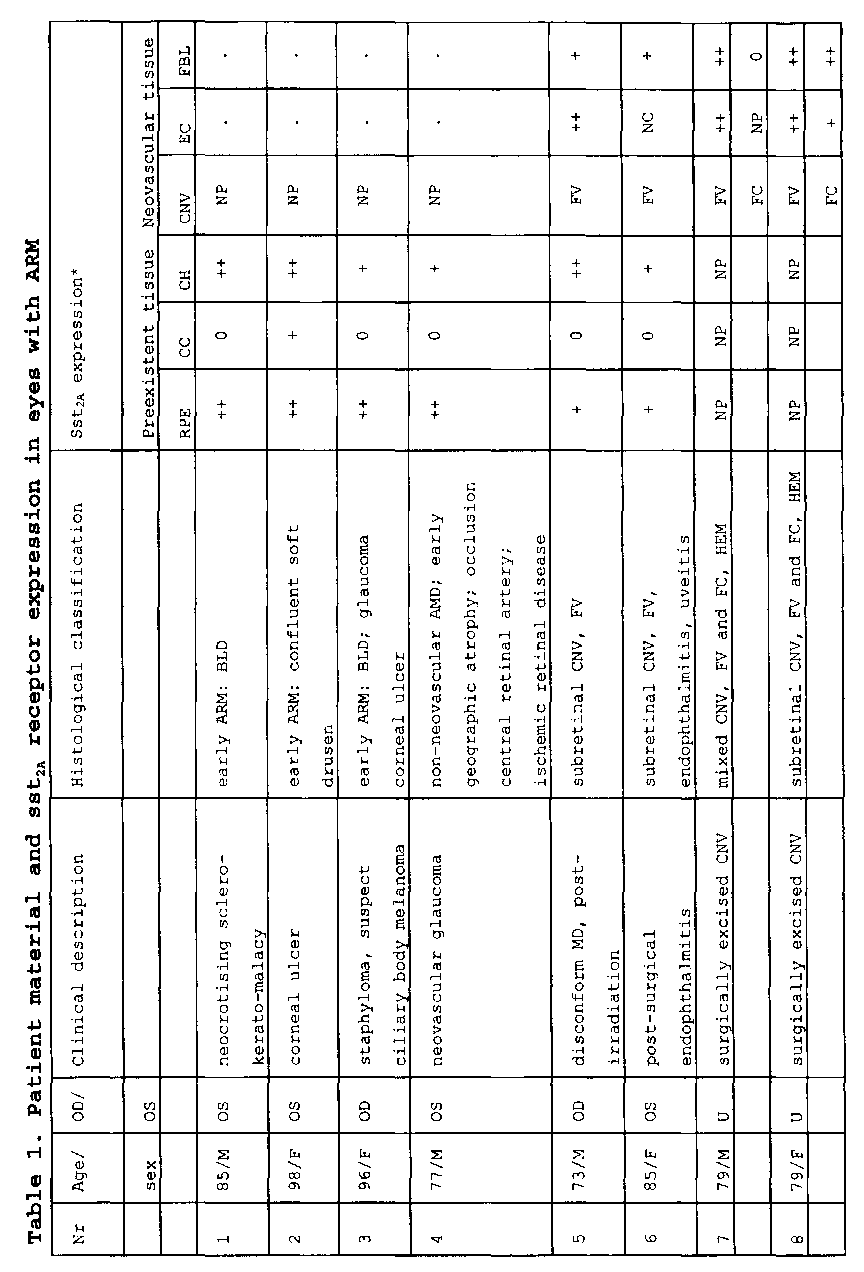

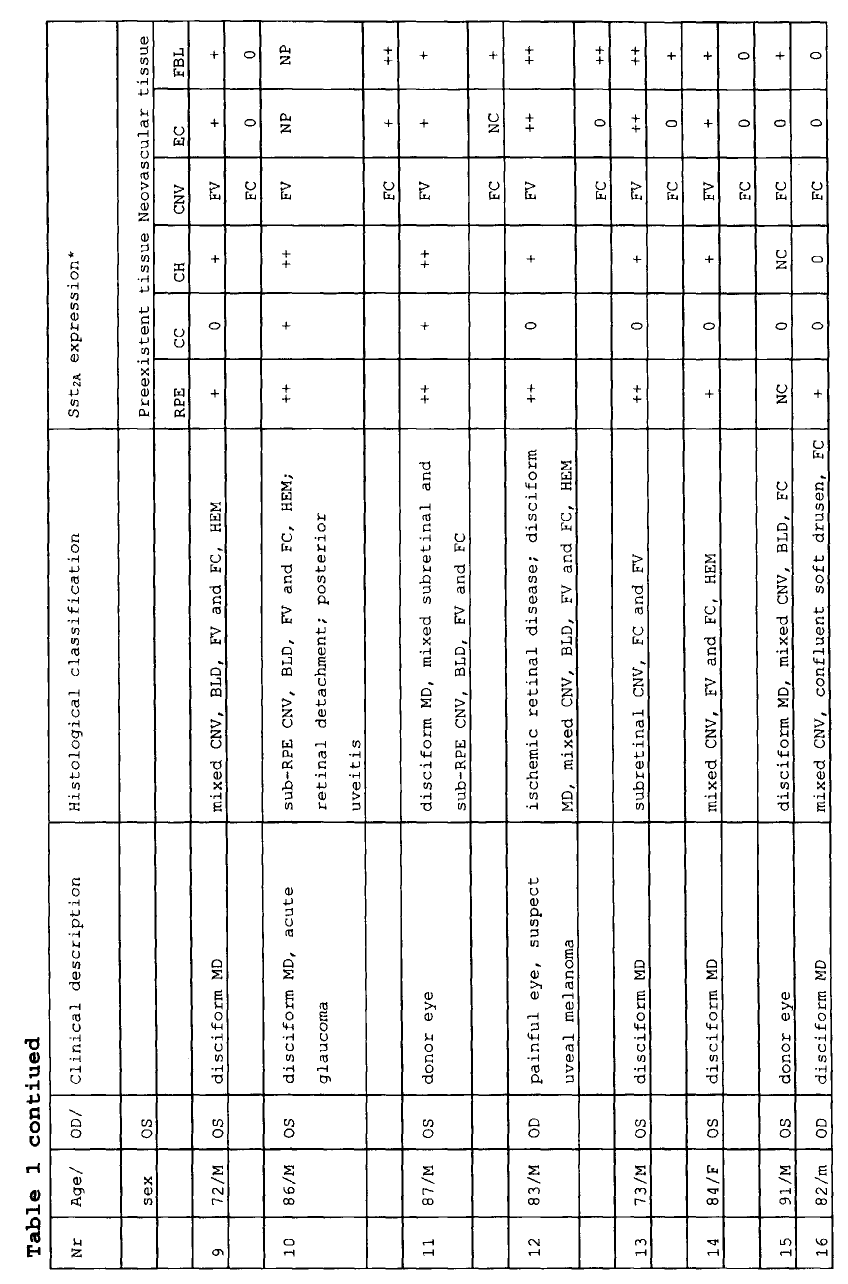

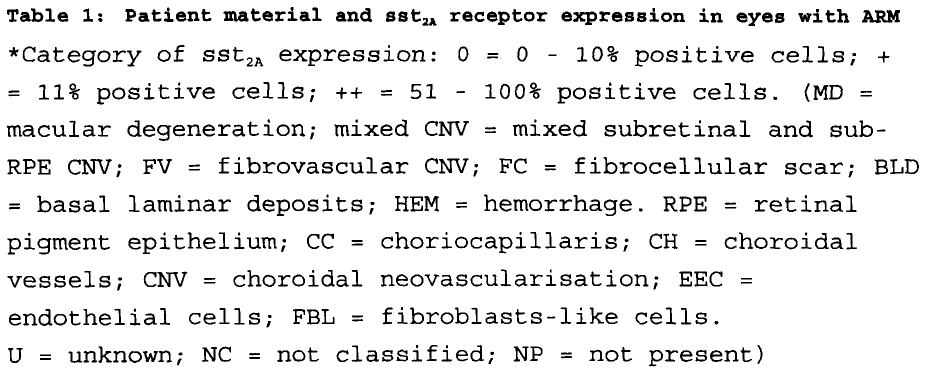

Eight eyes (of seven patients) were clinically diagnosed as AMD. In eight eyes, ARM was diagnosed histopathologically according to the following criteria. Early stages of ARM were characterized by the presence of basal laminar deposits, basal linear deposits (BLD), soft drusen and thickening of Bruch's membrane (n=3). Exudative AMD was classified as sub-RPE CNV, subretinal CNV (between neuroretina and RPE) or mixed sub-RPE and subretinal CNV (n=12). Photoreceptors, Bruch's membrane and BLD were helpful in the orientation of the specimens. Sub-RPE CNV and mixed CNV, or subretinal CNV in elderly patients in the presence of BLD or soft drusen were classified as CNV secondary to AMD. In CNV, we recorded the presence of fibrovascular or fibrocellular tissue, hemorrhage, vascular endothelium, BLD and RPE. On eye was classified as non-neovascular (geographic) AMD. Eight enucleated eyes without ARM (donor-eyes or enucleated for other reasons) were used as controls (Table 2).

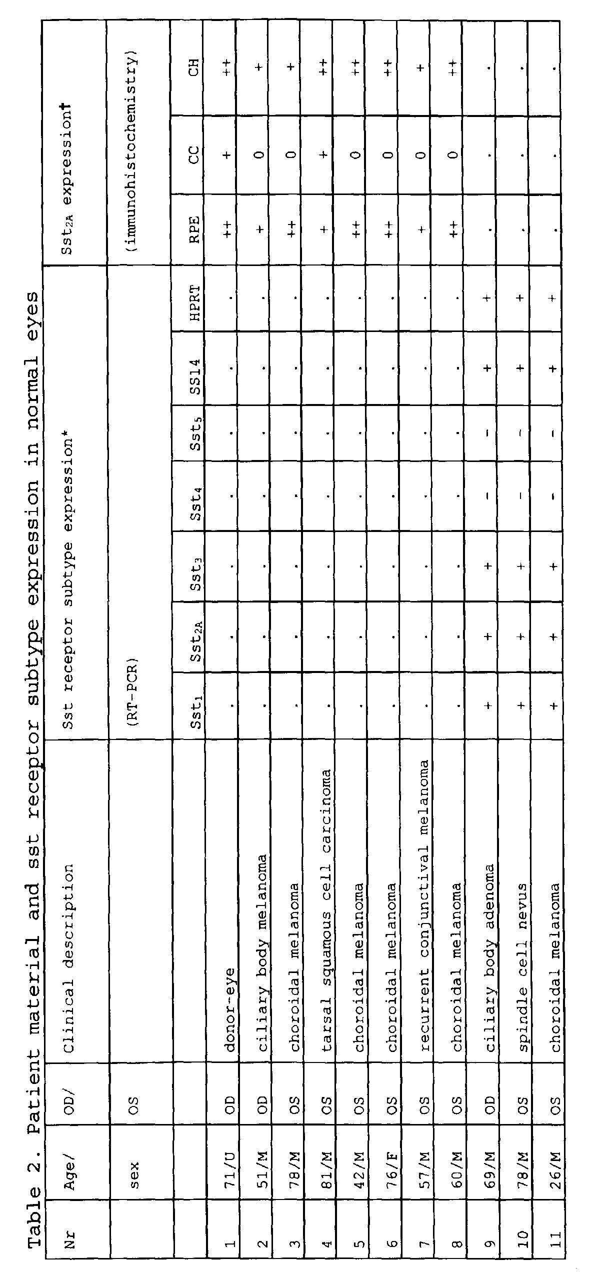

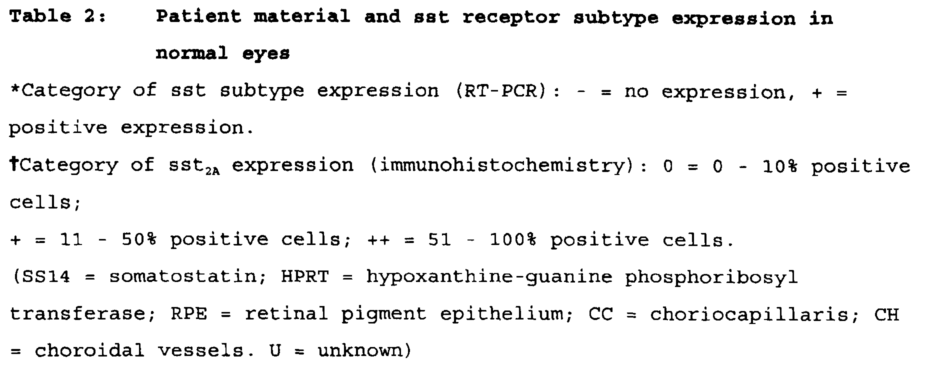

The eyes were processed for routine diagnostic procedures by fixation in formaldehyde and embedded in paraffin.

### Immunohistochemistry

Rabbit anti-human somatostatin receptor 2A polyclonal antibody was kindly provided by A. Schonbrunn, (Dept. of Pharmacology, University of Texas, Houston, USA). The sst_{2A} antibody was raised against a 22-aminoacid peptide located in the C-terminal region of the sst₂ receptor. The sst_{2A} antibody was characterized and tested before by Western blot analysis and peptide binding. Mouse monoclonal antibody against smooth muscle actin (sma) was obtained from Biogenex, San Ramon, CA, USA; mouse monoclonal antibody against macrophages (CD68) from Dako, Glastrup, Denmark. Five µm sections were prepared. The sections were dewaxed, rehydrated and (for sst_{2A} and CD68) microwave-heated for 10 minutes. After blocking with normal goat serum (Dako, 1:10) for 15 minutes, the slides were incubated with sst_{2A} antibody (1:1000) or CD68 antibody (1:2000) overnight at 4°C, or with anti-sma (1:150) for 1 hour at room temperature. The sections were further incubated biotinylated multilink antibodies for 30 minutes, followed by alkaline phosphatase-labeled antibiotin (both Biogenex) for 30 minutes. The bound antibodies were visualized by incubating the sections with New Fuchsin for 30 minutes in the dark. The slides were counterstained with Mayer's hematoxylin, mounted and examined by light microscopy. The sst_{2A} expression was graded in 3 catagories: 0 (0-10% positive cells), 1 (11-50% positive cells) and 2(51-100% positive cells). Negative controls for immunohistochemistry included 1) omission of the primary antibody, 2) use of an irrelevant antibody of the same isotype, and 3) preabsorbtion of the sst_{2A} antibodies with the immunizing receptor peptide for 4 hours, at a concentration of 3 µg/ml.

### RT-PCR

In order to study the mRNA expression of sst subtypes in normal human eyes, posterior poles from three eyes (Table 2) were dissected directly after enucleation. A sample of about 0.2 mm² localized in the macula, including RPE, choroid and sclera, was snap-frozen in liquid nitrogen. RT-PCR was performed as described by Ferone *et al*. ("In vitro characterization of somatostatin receptors in the human thymus and effects of somatostatin and octreotide on cultured thymic epithelial cells". Endocrinology, 1999;140:373-380) the nucleotide sequences of the primers used are given in Table 3.

**Table 3**

| Primers used for RT-PCR analysis | | | |
|---|---|---|---|
| receptor | primer | sequence (5'-3')* | product size (base pair) |
| sst₁ | forward | ATGGTGGCCCTCAAGGCCGG | 318 |
| | reverse | CGCGGTGGCGTAATAGTCAA | |
| sst_{2A} | forward | GCCAAGATGAAGACCATCAC | 414 |
| | reverse | GATGAACCCTGTGTACCAAGC | |
| sst₃ | forward | CCAACGTCTACATCCTCAACC | 314 |
| | reverse | TCCCGAGAAGACCACCAC | |
| sst₄ | forward | ATCTTCGCAGACACCAGACC | 321 |
| | reverse | ATCAAGGCTGGTCACGACGA | |
| sst₅ | forward | CGTCTTCATCATCTACACGG | 226 |
| | reverse | CCGTCTTCATCATCTACACGG | |
| SS14 | forward | GATGCTGTCCTGCCGCCTCCAG | 349 |
| | reverse | ACAGGATGTGAAAGTCTTCCA | |
| HPRT | forward | CAGGACTGAACGTCTTGCTC | 413 |
| | reverse | CAAATCCAACAAAGTCTGGC | |
| **Table 3: Primers used for RT-PCR analysis** The sequences of the primers for sst₁ were derived and adapted from Wulfsen *et al*., for sst₅ from Kubota *et al*., and all others were designated by use of the Primer3!software (http://www-gemome.wi.mit.edu/genome_software/other/primer3.html) and the appropriate GenBank entries. (SS14 = somatostatin; HPRT = hypoxanthine-guanine phosphoribosyl transferase) | | | |

Several controls were included in the RT-PCR experiments. To ascertain that no detectable genomic DNA was present in the polyA⁺ mRNA preparation (since the sst genes are intron-less), the cDNA reactions were also performed without reverse transcriptase and amplified with each primer-pair. Amplification of the cDNA samples with the HPRT (hypoxanthine-guanine phosphoribosyl transferase) specific primers served as positive control for the quality of the cDNA. To exclude contamination of the PCR reaction mixtures, the reactions were also performed in the absence of DNA template in parallel with cDNA samples. As a positive control for the PCR reactions of the sst receptor subtypes, 0.1 to 0.001 µg of human genomic DNA, representing approximately 30.000 to 300 copies of sst-template, was amplified in parallel with the cDNA samples. As a positive control for the PCR of the HPRT gene and somatostatin cDNA aliquots of a cDNA sample known to contain somatostatin and HPRT mRNA were amplified, because these primer-pairs did enclose introns in the genomic DNA.

### Results

In normal retina (n=8) high sst_{2A} expression was found in the inner plexiform layer (IPL) and moderate expression was found in the outer plexiform layer (OPL), the cellular membrane of the inner nuclear layer (INL) (Figure 1A), and the RPE. RPE stained most frequently at the apical side in a membranous pattern (Figure 1B), which was also noted in tangentially cut sections. Thick walled choroidal vessels stained mostly positive, whereas choriocapillaris stained only sporadically (Table 1). In negative controls, no staining was detected.

In the eyes with early ARM (n=3), sst_{2A} expression of the neuroretina, choroidal vessels and choriocapillaris was similar to normal controls (Table 1). The RPE stained positive in all cases, BLD were negative (Figure 1C).

In eyes with exudative AMD (n-12) Bruch's membrane and BLD did not show sst_{2A} expression (Table 1). The choriocapillaris showed focal expression in only two eyes. Approximately 50-75% of choroidal arteries stained positive, which was similar to normal controls. The CNV, both surgically excised and in enucleated eyes, could be subdivided in three groups, regarding the activity of neovascularization: the first group consisted of fibrovascular tissue with inflammatory cells, fibroblast-like cells and sparse fibrosis (n=2). The second group consisted of fibrocellular scar tissue (n=2), and the third group consisted of a mixture of both fibrovascular and fibrocellular tissue (n=8).

In the CNV monolayers of pigmented cells adjacent to BLD were scored as RPE cells. About half of these morphologically RPE cells showed sst_{2A} expression (Figure 1D). The expression of sst_{2A} in newly formed endothelial cells was upregulated in fibrovascular membranes. Similarly, sst_{2A} was upregulated in endothelial cells of mixed fibrovascular membranes (Figure 1E). Fibroblast-like cells and macrophages stained highly positive in young membranes, less strongly in elder scars (Figure 1D and E). Little or negative staining was observed in old hypocellular scars (Figure 1F). Peptide blocking significantly decreased staining of all structures mentioned (Figure 1G).

In one eye with a mixed fibrovascular and fibrocellular membrane (eye number 12), we found positive staining of myofibroblasts in a hypercellular area of the underlying choroid in the posterior pole. This area also stained positive with antibodies directed against sma and CD68, confirming the presence of myofibroblastic cells and macrophages.

In the eye with non-neovascular AMD, the staining pattern was similar to control tissue. The RPE stained positive. No staining was seen in the choriocapillaris, and vessels in the choroid were mostly positive.

RT-PCR analysis of 3 posterior poles, including retina, RPE, choroid and sclera, revealed that mRNA encoding for sst₁, sst_{2A}, sst₃ and somatostatin is expressed in the posterior pole of normal human eye. No mRNA encoding for sst₄ or sst₅ could be detected (Figure 2, Table 2).

From this Example it follows that normal human eyes and eyes with early and late stages of ARM express sst_{2A}. The localization of sst_{2A} expression in the neuroretina physiological neuromodulator-function of somatostatin. In early stages of ARM, the choroidal vasculature and neuroretinal tissue stained identical to control tissue. We found no expression of sst_{2A} in BLD or drusen, which is in contrast with findings for other angiogenic growth factors like VEGF.

In eyes with exudative AMD, we found increased expression of sst_{2A} in endothelial cells and fibroblast-like cells in early CNV. The expression of sst_{2A} in newly formed capillaries was abundant in fibrovascular CNV membranes. Similarly, in the active component of mixed fibrovascular/fibrocellular CNV, sst_{2A} was highly expressed in endothelial cells.

The angiogenic cells of CNV membranes are capable of receiving angiogenic inhibition, directly receptor mediated or indirectly via inhibition of GH and IGF-I by somatostatin.

Furthermore, strong positive sst_{2A} expression in fibroblast-like cells and macrophages in fibrovascular CNV and in intrachoroidal myofibroblasts is found. Sst_{2A} staining in myofibroblasts may be due to cross-reactivity to myosin (See: Reubi JC, Laissue JA, Waser B, *et al*., "Immunohistochemical detection of somatostatin sst_{2A} receptors in the lymphatic, smooth muscular, and peripheral nervous systems of the human gastrointestinal tract: fact and artifacts", *J Chin Endocrinol Metab*. 1999;84:2942-2950), but macrophages have been shown to express sst_{2A} (See: Ten Bokum AMC, Hofland LJ, de Jong G, *et al*. "Immunohistochemical localization of somatostatin receptor sst_{2A} in sarcoid granulomas", *Eur J Clin Invest*. 1999;29:630-636).

In the overlying neuroretina of eyes with CNV, no obvious change of sst_{2A} expression and localization in comparison to normal eyes was observed. This is in contrast to VEGF expression in neuronal tissue, which is upregulated under hypoxic circumstances (See: Kliffen M, Sharma HS, Mooy CM, *et al*. "Increased expression of angiogenic growth factors in age-related maculopathy", *Br J Ophthalmol*. 1997;81:154-162 and Pe'er J, Shweiki D, Itin A, *et al*. "Hypoxia-induced espression of vascular endothelial growth factor by retinal cells is a common factor in neovascularizing ocular diseases", *Laboratory Investigation*. 1995;72:638-644). This indicates that the function of somatostatin on neuronal tissue is not influenced by hypoxic retinal disease.

From these results it follows that somatostatin and VEGF have distinct functions in the control of angiogenesis.

Local synthesis of sst_{2A} in the macula of normal human eyes was confirmed with RT-PCR. Also, the expression of mRNA encoding for sst subtypes 1 and 3 was demonstrated.

mRNA expression of the neuropeptide somatostatin in the human macula was observed. The production of both somatostatin and its receptors simultaneously suggests an autocrine action of somatostatin I the human retina, which finding is used in the medicament of the present invention.

## Claims

1. Use of a compound that binds to at least one somatostatin receptor in the eye in the manufacture of a medicament for the treatment of a choroidal neovascularization (CNV) related disorder.

2. Use according to claim 1, wherein said compound binds to a somatostatin 2A receptor (sst_{2A}).

3. Use according to any of the previous claims, wherein the CNV related disease is exudative age-related macular degeneration (AMD).

4. Use according to any of the previous claims, wherein said compound is selected from the group consisting of somatostatin and somatostatin analogues.

5. Use according to claim 4, wherein the somatostatin analogue is selected from the group consisting of octreotide, vapreotide and lanreotide.

6. Use according to any of the previous claims, wherein said medicament is formulated for topical administration.

7. Use according to any of the previous claims, wherein said medicament is in the form selected from the group consisting of eye drops, eye gel and eye ointment.

8. Use according to any of claims 1-5, wherein said medicament is formulated for subcutaneous or intramuscular administration, preferably in slow release form.

9. A method for treating choroidal neovascularization or disorder related or associated therewith, comprising administering a compound that binds to at least one somatostatin receptor in the eye.

10. The method of claim 9, wherein the said compound is administered on or in the eye.
